# EUROPEAN PATENT APPLICATION

(11) **EP 1 405 558 A1**
(43) Date of publication of application: **07.04.2004**
(21) Application number: 02743817.5
(22) Date of filing: 04.07.2002
(51) Int. Cl.: A01H 5/00, C12N 9/10

(54) **METHOD OF ELEVATING GLUTAMIC ACID CONTENT IN PLANT AND PLANTS HAVING ELEVATED GLUTAMIC ACID CONTENT**

(30) Priority: 09.07.2001 JP 2001208238
(71) Applicant: Ajinomoto Co., Inc., Tokyo 104-0031 (JP); Kazusa Dna Research Institute, Kisarazu-shi, Chiba 292-0818 (JP)
(72) Inventor: IGARASHI, D., C. Res. Lab. Ajinomoto Co., Inc., Kawasaki-shi, Kanagawa 210-0801 (JP); OHSUMI, C., C. Res. Lab .Ajinomoto Co., Inc., Kawasaki-shi, Kanagawa 210-0801 (JP)
(74) Representative: Strehl Schübel-Hopf & Partner
(86) International application number: PCT/JP2002/006766
(87) International publication number: WO 2003/005809

(57) **Abstract**

The present invention provides a method for increasing glutamate content of plants and/or seeds by deleting or reducing GGT activity, and also plants and/or seeds having deleted or reduced GGT activity, particularly having a glutamate content higher than that of corresponding wild type plants cultivated under the same conditions.

SEQUENCE LISTING

<110> Ajinomoto Co., Inc.
<110> Kazusa DNA Research Institute

<120> Methods of increasing glutamic acid content in plants

<130> EPA-63319

<140>
<141>

<150> JP 2001-208238
<151> 2001-07-09

<160> 20

<170> PatentIn Ver. 2.1

<210> 1
<211> 481
<212> PRT
<213> Arabidopsis thaliana

<400> 1
Met Ala Leu Lys Ala Leu Asp Tyr Asp Thr Leu Asn Glu Asn Val Lys
1               5                   10                  15

Lys Cys Gln Tyr Ala Val Arg Gly Glu Leu Tyr Leu Arg Ala Ser Glu

            20                  25                  30
Leu Gln Lys Glu Gly Lys Lys Val Ile Phe Thr Asn Val Gly Asn Pro
        35                  40                  45
His Ala Leu Gly Gln Lys Pro Leu Thr Phe Pro Arg Gln Val Val Ala
    50                  55                  60
Leu Cys Gln Ala Pro Phe Leu Leu Asp Asp Pro Asn Val Gly Met Leu
65                  70                  75                  80
Phe Pro Ala Asp Ala Ile Ala Arg Ala Lys His Tyr Leu Ser Leu Thr
                85                  90                  95
Ser Gly Gly Leu Gly Ala Tyr Ser Asp Ser Arg Gly Leu Pro Gly Val
            100                 105                 110
Arg Lys Glu Val Ala Glu Phe Ile Gln Arg Arg Asp Gly Tyr Pro Ser
        115                 120                 125
Asp Pro Glu Leu Ile Phe Leu Thr Asp Gly Ala Ser Lys Gly Val Met
    130                 135                 140
Gln Ile Leu Asn Cys Val Ile Arg Gly Asn Gly Asp Gly Ile Leu Val
145                 150                 155                 160
Pro Val Pro Gln Tyr Pro Leu Tyr Ser Ala Thr Ile Ser Leu Leu Gly
                165                 170                 175
Gly Thr Leu Val Pro Tyr Tyr Leu Asp Glu Ser Glu Asn Trp Gly Leu

            180                 185                 190

Asp Val Ala Asn Leu Arg Gln Ser Val Ala Gln Ala Arg Ser Gln Gly
        195                 200                 205

Ile Thr Val Arg Ala Met Val Ile Ile Asn Pro Gly Asn Pro Thr Gly
    210                 215                 220

Gln Cys Leu Ser Glu Ala Asn Ile Arg Glu Ile Leu Lys Phe Cys Tyr
225                 230                 235                 240

Asn Glu Lys Leu Val Leu Leu Gly Asp Glu Val Tyr Gln Gln Asn Ile
                245                 250                 255

Tyr Gln Asp Glu Arg Pro Phe Ile Ser Ser Lys Lys Val Leu Met Glu
            260                 265                 270

Met Gly Ser Pro Phe Ser Lys Glu Val Gln Leu Val Ser Phe His Thr
        275                 280                 285

Val Ser Lys Gly Tyr Trp Gly Glu Cys Gly Gln Arg Gly Gly Tyr Phe
    290                 295                 300

Glu Met Thr Asn Leu Pro Pro Arg Val Val Glu Glu Ile Tyr Lys Val
305                 310                 315                 320

Ala Ser Ile Ala Leu Ser Pro Asn Val Ser Ala Gln Ile Phe Met Gly
                325                 330                 335

Leu Met Val Asn Pro Pro Lys Pro Gly Asp Ile Ser Tyr Asp Gln Phe

            340                 345                 350

Ala Arg Glu Ser Lys Gly Ile Leu Glu Ser Leu Arg Arg Arg Ala Arg
        355                 360                 365

Leu Met Thr Asp Gly Phe Asn Ser Cys Lys Asn Val Val Cys Asn Phe
    370                 375                 380

Thr Glu Gly Ala Met Tyr Ser Phe Pro Gln Ile Arg Leu Pro Thr Gly
385                 390                 395                 400

Ala Leu Gln Ala Ala Lys Gln Ala Gly Lys Val Pro Asp Val Phe Tyr
                405                 410                 415

Cys Leu Lys Leu Leu Glu Ala Thr Gly Ile Ser Thr Val Pro Gly Ser
            420                 425                 430

Gly Phe Gly Gln Lys Glu Gly Val Phe His Leu Arg Thr Thr Ile Leu
        435                 440                 445

Pro Ala Glu Asp Glu Met Pro Glu Ile Met Asp Ser Phe Lys Lys Phe
    450                 455                 460

Asn Asp Glu Phe Met Thr Gln Tyr Asp Asn Asn Phe Gly Tyr Ser Lys
465                 470                 475                 480

Met

<210> 2
<211> 26
<212> DNA
<213> Artificial Sequence

<220>
<223> Description of Artificial Sequence:PCR primer

<400> 2
ctctagaacc gaacgtgact ctccag 26

<210> 3
<211> 25
<212> DNA
<213> Artificial Sequence

<220>
<223> Description of Artificial Sequence:PCR primer

<400> 3
ccatgatctc cggcatctca tcttc 25

<210> 4
<211> 26
<212> DNA
<213> Artificial Sequence

<220>

<223> Description of Artificial Sequence:PCR primer

<400> 4
atcacaaatc aggcacaagg ttagac 26

<210> 5
<211> 26
<212> DNA
<213> Artificial Sequence

<220>
<223> Description of Artificial Sequence:PCR primer

<400> 5
ggagggaaga agtgagctag ggattg 26

<210> 6
<211> 26
<212> DNA
<213> Artificial Sequence

<220>
<223> Description of Artificial Sequence:PCR primer

<400> 6
cgctcatcct ggtatatgtt ctgctg 26

<210> 7
<211> 20
<212> DNA
<213> Artificial Sequence

<220>
<223> Description of Artificial Sequence:PCR primer

<400> 7
ataacgctgc ggacatctac 20

<210> 8
<211> 24
<212> DNA
<213> Artificial Sequence

<220>
<223> Description of Artificial Sequence:PCR primer

<400> 8
ttagacaagt atctttcgga tgtg 24

<210> 9
<211> 25
<212> DNA
<213> Artificial Sequence

<220>

<223> Description of Artificial Sequence:PCR primer

<400> 9
aacgctgcgg acatctacat ttttg 25

<210> 10
<211> 30
<212> DNA
<213> Artificial Sequence

<220>
<223> Description of Artificial Sequence:PCR primer

<400> 10
gtgggttaat taagaattca gtacattaaa 30

<210> 11
<211> 25
<212> DNA
<213> Artificial Sequence

<220>
<223> Description of Artificial Sequence:PCR primer

<400> 11
aagaaaatgc cgatacttca ttggc 25

<210> 12
<211> 25
<212> DNA
<213> Artificial Sequence

<220>
<223> Description of Artificial Sequence:PCR primer

<400> 12
aagaaaatgc cgatacttca ttggc 25

<210> 13
<211> 20
<212> DNA
<213> Artificial Sequence

<220>
<223> Description of Artificial Sequence:PCR primer

<400> 13
tagatccgaa actatcagtg 20

<210> 14
<211> 26
<212> DNA
<213> Artificial Sequence

<220>

<223> Description of Artificial Sequence:PCR primer

<400> 14
acgtgactcc ctttaattct ccgctc 26

<210> 15
<211> 25
<212> DNA
<213> Artificial Sequence

<220>
<223> Description of Artificial Sequence:PCR primer

<400> 15
cctaactttt ggtgtgatga tgctg 25

<210> 16
<211> 25
<212> DNA
<213> Artificial Sequence

<220>
<223> Description of Artificial Sequence:PCR primer

<400> 16
ttcccttaat tctccgctca tgatc 25

<210> 17
<211> 25
<212> DNA
<213> Artificial Sequence

<220>
<223> Description of Artificial Sequence:PCR primer

<400> 17
ttcccttaat tctccgctca tgatc 25

<210> 18
<211> 25
<212> DNA
<213> Artificial Sequence

<220>
<223> Description of Artificial Sequence:PCR primer

<400> 18
ttcccttaat tctccgctca tgatc 25

<210> 19
<211> 27
<212> DNA
<213> Artificial Sequence

<223> Description of Artificial Sequence:PCR primer

<400> 19
gtttcacatc aacattgtgg tcattgg 27

<210> 20
<211> 24
<212> DNA
<213> Artificial Sequence

<220>
<223> Description of Artificial Sequence:PCR primer

<400> 20
gagtacttgg gggtagtggc atcc 24

## Description

### Background of the Invention

The present invention relates to a method for increasing glutamate content of a plant and/or a seed, a plant and/or a seed having an increased glutamate content, use of a plant and/or a seed having an increased glutamate content for the production of foods, and a food containing a plant and/or a seed having an increased glutamate content.

In general, glutamate is commonly present in proteins and is known as a tasty ingredient contained in tomatoes, soy beans, adzuki beans, broad beans, kidney beans, peas, etc. and also in foods made by the fermentation of soy beans or the like. Glutamate is synthesized in the early stage of the biosynthesis of amino acids in higher plants, and it serves as an amino group donor for major amino acids such as alanine, glycine, serine, proline and arginine. Glutamine and asparagine synthesized in leaves are carried into seeds through sieve tubes and used for synthesizing the above-described major amino acids after glutamate is synthesized. Enzymes which transfer the amino group of glutamine are believed to function in the compartmentalized tissues such as a seed coat, an embryosac fluid and a cotyledon based on the role of each function which is strictly regulated. Thus it has not been easy to increase free glutamate content in plants because glutamate is the donor of an amino group for various reactions occurring in vivo including protein synthesis and is metabolized through biosynthesis pathways which are diversely and complicatedly regulated. Particularly it is considered to be difficult to increase in amount of a specified amino acid in its free form because amino acids are stored mainly in the form of protein. In fact, only several reports on these facts are known until now. The reports include, for example, a report that free glutamate content of roots of tobacco and corn was increased by the introduction of glutamate dehydrogenase genes, and a report that free lysine accumulated in soybean seeds when a gene encoding a protein having a high lysine content was excessively expressed in leaves.

Alanine aminotransferase is known as one of enzymes concerning the metabolism of glutamate. This enzyme catalyzes a reaction of transferring amino group of L-alanine into α-ketoglutarate and the reverse reaction thereof. This enzyme constitutes a part of the above-described glutamate metabolism. It is also known that this enzyme has an activity of catalyzing reactions of transferring amino group from alanine to glyoxylate and also from glutamate to glyoxylate for synthesizing glycine (Biochem. J. 195:235-239, 1981). On the other hand, the possibility is suggested that alanine aminotransferase existing in peroxisomes has glutamate glyoxylate aminotransferase activity, namely, an activity of synthesizing α-ketoglutarate and glycine using glutamate and glyoxylate as the substrates [Noguchi T. and HAYASHI S., Biochem. J. 195-235-239 (1981); Orzechowski et al., Acta Biochem. Pol.: 447-457 (1999) and Orzechowski et al., Acta Physiol. Plant 21: 331-334 (1999)]. However, the roles of alanine aminotransferase and glutamate glyoxylate aminotransferase activity on the increase or decrease of glutamate content of plants has not been elucidated and there is no report regarding the possibility that the glutamate content in plants or seed could be actually increased by genetic manipulation of the genes encoding proteins having alanine aminotransferase or glutamate glyoxylate aminotransferase activity.

### Summary of the Invention

The object of the present invention is to provide a method for increasing glutamate content of a plant and/or a seed, a plant and/or a seed having an increased glutamate content, use of a plant and/or a seed having an increased glutamate content for the production of a food, and a food containing a plant and/or a seed having an increased glutamate content.

The inventors have found that glutamate content of plants and/or seeds can be increased by inhibiting glutamate glyoxylate aminotransferase (GGT) activity. The present invention has been completed on the basis of this finding.

Namely, the present invention provides a method for increasing glutamate content of plants and/or seeds by lacking or reducing GGT activity, and the plants and/or seeds wherein a GGT activity is lacked or reduced and particularly plants and/or seeds having a glutamate content higher than that of corresponding wild type plants cultured under the same conditions.

In particular, the present invention relates to a method for increasing glutamate content of plants and/or seeds by lacking or reducing GGT activity by inhibiting the function of genes encoding a protein having GGT activity, and the plants having lacked or reduced GGT activity, in particular, plants and seeds having lacked or reduced GGT activity and having glutamate content higher than that of corresponding wild type plants cultured under the same conditions.

### Brief Description of the Drawings

Fig. 1 shows the comparison of amino acid sequences of alanine aminotransferase (AlaAT: the same as GGT) from Arabidopsis thaliana. The asterisks show the position where all the amino acids are identical.
Fig. 2 shows the location of inserted tag. (A) The scheme of AlaAT1 genome structure and the location of inserted tag. The boxes show exon and the line shows intron. (B) The nucleotide sequence and amino acid sequence of the wild type (WT) are shown on the top, and the nucleotide sequence wherein a T-DNA is inserted at around and its amino acid sequence of line 8046 are shown on the bottom. The boxes show the region replaced by the insertion of T-DNA.
Fig.3 (A) The figure indicating the location of primers which were used for the selection of homozygotes. (B) The segregation ratio of homozygotes and heterozygotes was shown.
Fig. 4 shows the AlaAT1 gene expression at the mRNA level by RT-PCR. WT: wild type, 8046: homozygotic AlaAT1 tag-inserted line.
Fig. 5 shows the enzyme activity in tag-inserted line aat1-1. Ala+αKG, Glu+Pyr, and Glu+glyoxylate indicate the activities of catalyzing the reaction of Ala+αKG → Glu+Pyr, Glu+Pyr → Ala+ αKG and Glu+glyoxylate → Gly+ αKG, respectively. The activities are indicated as the change in absorbance (ΔA) at 340nm per one minute per 1µg of protein, coupled with the oxidation reaction of NADH.
Fig. 6 shows the scheme of photorespiration pathway in higher plants. An arrow indicates the reaction which glutamate glyoxylate aminotransferase catalyzes.
Fig. 7 shows the amino acids content of seedlings cultivated for two weeks on PNS medium containing 1% sucrose. A: amino acids contents per fresh weight, B: the ratio to total amino acid. Control: wild type plant, aat1-1: line 8046, the AlaAT1 tag-inserted plant.
Fig. 8 shows the amino acids content (nmol/mg FW) in the seeds of aat1-1.
Fig.9 shows the relative amino acids content (%) in the seeds of aat1-1.

### Description of the Preferred Embodiments

The object of the present invention is to increase glutamate content of plants and/or seeds. This object can be attained by removing or reducing GGT activity.

In one embodiment of the present invention, the functions of a gene encoding GGT are inhibited. The term "the function of a gene encoding glutamate glyoxylate aminotransferase (or GGT)" as used herein indicates the function of a gene to express glutamate glyoxylate aminotransferase having the activity of wild type glutamate glyoxylate aminotransferase. Accordingly, an expression "the function of a gene encoding glutamate glyoxylate aminotransferase (or GGT) is inhibited" involves, for example, a case wherein the gene itself is disrupted, a case wherein the expression of the gene is inhibited at the transcription or translation level and a case wherein the gene is modified and, as a result, the expressed protein has no activity of the wild type GGT.

The deletion or reduction of GGT activity by inhibiting the function of the genes encoding GGT can be attained by, for example, disrupting GGT genes or transforming the plant with genetic constructs for inhibiting the expression.

The object of the present invention can be attained by lacking or reducing GGT activity as described above. The enzymatic activity may be deleted or reduced at any of the transcriptional level, translational level and protein level. For example, the plant having the lowered activity may be screened by acclimating the plant to various growing conditions and determining GGT activity using a method which will be described below. It is also possible to delete or to reduce the activity by modifying or disrupting the genes encoding GGT in the plant genome. It is also possible to remove or to reduce GGT activity by antisense or cosuppression method. Particularly in the present invention, from the viewpoint of the stability of the properties, disruption of GGT genes is preferred. Such a gene disruption can be achieved by the insertion of a transposon, insertion of T-DNA or mutagenesis by the treatment with a mutagen such as EMS. The ordinary techniques of gene disruption by the insertion of transposon or T-DNA or the treatment with the mutagen are well known by those skilled in the art. When a prepared library of gene-disrupted plants is available, desired transformed plants can be easily obtained by screening the library to find the plants containing disrupted GGT genes. Such a library is commercially available.

As compared with the wild type plants grown under the same conditions, the plants of the present invention have not more than about 80 %, preferably not more than about 50 % and more preferably not more than 30 %, of GGT activity at some stage of the development, in any of the total protein, fresh weight or a leaf.

The deletion or reduction of GGT activity in the present invention preferably occurs in a peroxisome, particularly in a peroxisome in a photosynthesis tissue. The photosynthesis tissue may be any tissue conducting the photosynthesis under ordinary cultivation conditions, such as leaves, stems and siliques.

Thus, as compared with wild type plants grown under the same conditions, the plants having a glutamate content increased by the method of the present invention have not more than about 80 %, preferably not more than about 50 % and more preferably not more than 30 %, of the GGT activity in the total protein extracted preferably from the photosynthesis tissues, more preferably from a peroxisome in the photosynthesis tissue, of the GGT activity in tissue or in the intracellular organs per fresh weight or of the GGT activity in the tissue or the intracellular organs per leaf.

The term "glutamate glyoxylate aminotransferase" as used herein indicates a protein having glutamate glyoxylate aminotransferase activity, which may be also abbreviated to "GGT". The term "genes encoding glutamate glyoxylate aminotransferase" herein involves all the nucleic acid fragments encoding a protein having the glutamate glyoxylate aminotransferase activity.

The GGT genes used as the target in the present invention can also be obtained from plants. For example, DNA base sequence information of GGT genes can be obtained by retrieving it from a database using "alanine aminotransferase" as a keyword. According to the sequence information, the full-length cDNA can be obtained by RT-PCR, 5'-RACE and 3'-RACE. It is also possible to obtain the cDNA by screening cDNA library by hybridization with a suitable probe according to the known sequence information. The probes used for the screening can be prepared according to the amino acid or DNA sequence of GGT.

In the present invention, it is preferred that the interested GGT is localized in a peroxisome, particularly a peroxisome in the photosynthesis tissues as described above. The localization of GGT in the peroxisome can be deduced from the presence of N-terminal sequence or C terminal sequence characteristic to protein localized in the peroxisome. It is also possible to confirm the localization by fusing a reporter gene such as GFP or GUS to GGT gene while keeping the localization in the peroxisome, expressing GGT in the cell and determining it. In another method, the localization can be confirmed by detection of expressed tagged-GGT with a specific antibody.

When a gene-disrupted plant library or the like is available, the plants of the present invention can be obtained by screening GGT gene-disrupted plants in the library. By suitable combination of the primers and probes, it is also possible to confirm how the GGT genes are disrupted in those plants. The screening method and analysis methods are well known in the art, and one can refer to, for example, *"Shokubutsu no Genome Kenkyu Protocol* (Protocol of Study of Plant genome)" (published by Shujunsha). Even when such a library is unavailable, by using genetic engineering methods such as transposon and T-DNA, it is possible to obtain such gene-disrupted plants, particularly plants having disrupted GGT genes or plants defective or inhibitive in GGT activity by some other reasons.

Nucleic acid constructs usable for producing the gene-disrupted plants in an embodiment of the present invention can be prepared by a method well known in the art. A molecular biological means including the procedures of designing nucleic acid constructs; isolating them and determining the sequence thereof can be found in literatures such as Sambrook et al., Molecular cloning-Laboratory manual, Edition 2, Cold Spring Harbor Laboratory Press. For preparing the nucleic acid constructs usable in the present invention, gene amplification by PCR method may be required in some cases. As for the PCR method, for example, F. M. Ausubel et al. (eds), Current Protocols in Molecular Biology, John Wiley & Sons, Inc. (1994) can be referred to.

The gene disruption in plants can be conducted by a method generally known in the art. For example, genomic GGT gene may be preferably disrupted as the target using a transposon, or T-DNA. The design of the genetic construct suitable for this method will be easily understood by those skilled in the art. The general techniques of the gene disruption with the transposon or T-DNA are described in, for example, Plant molecular biology manual 2^{nd}, S. B. Gelvin and R. A. Schilper.

The method for introducing the nucleic acid construct in the above-described embodiment is not particularly limited. Any method for introducing genes into plant cells or into plant bodies, known by those skilled in the art, can be selected depending on the hosts. For example, Agrobacterium mediated gene introduction method, the electroporation method or a particle gun can be employed. When Agrobacterium is used, the sequence to be introduced is preferably inserted between the left and right T-DNA border sequences. The suitable design and construction of the transformation vector thus based on T-DNA are well known in the art. Further, the conditions required for the infection of a specified plant with Agrobacterium having such a nucleic acid construct are also well known in the art. As for such techniques and conditions, Cell Technology, additional volume, "*Model Shokubutsu no Jikken Protocol; Ine*, *Shiroinunazuna Hen* (Experiment Protocol for Model Plants; Edition of Rice Plants and Arabidopsis thaliana) published by Shujunsha (1996) can be referred to.

Although the species of plants to be subjected to the gene manipulation are not particularly limited, plants species are preferred that can be easily cultivated and transformed and the regeneration system of which has been established. In addition to the plants having the above-described characteristic properties, species of plants, for which a large-scale cultivation technique has been established and which have a high utility value as foods, are preferred in the present invention. Those plants include, in addition to Arabidopsis thaliana as the model plant, rice plants, spinach, cabbages, lettuces, salads, celeries, cucumbers, tomatoes, broad beans, soybeans, adzuki beans, kidney beans and peas.

Then the transformants are selected from the genetically manipulated plant cells and the like thus obtained. The selection may also be based on the expression of marker genes present on the nucleic acid construct used for the transformation. For example, when the marker genes are drug resistant genes, the selection can be conducted by culturing or growing plant cells manipulated on a culture medium containing a suitable concentration of an antibiotic or a herbicide. When the marker genes are β-glucuronidase genes or luciferase genes, the transformants can be selected by screening for the activity. From thus identified transformants such as protoplasts, calli and explants, the plant bodies can be regenerated. A method known by those skilled in the art for each host plant can be employed for the regeneration. The plants thus obtained can be cultured by an ordinary method or, in other words, under the same conditions as those for the untransformed plants or under conditions suitable for the respective transformants. For the identification of the transformed plants containing the nucleic acid constructs of the present invention, various molecular biological methods can be employed in addition to the above-described marker gene selection method. In particular, the plants of the present invention having reduced GGT activity are preferably kept from a strong light so as to protect them from the growth inhibition.

For detecting the disruption of the target genes or the presence or absence of recombinant DNA inserts and the structures thereof, Southern hybridization, PCR, Northern blotting or RT-PCR can be employed.

Then the amount of the GGT protein, the GGT activity and the amount of mRNA of GGT of the thus obtained transformed plants can be determined. For example, the amount of the protein can be determined by Western blotting method or the like, and the amount of the mRNA can be determined by Northern blotting method, quantitative RT-TCR method or the like. GGT activity can be determined by an ordinary method (Plant Physiol. 99: 1520-1525). For example, GGT activity in a photosynthetic tissue can be determined by freeze-drying the photosynthetic tissue of a plant such as leaves with liquid nitrogen, pulverizing the frozen tissue, suspending the obtained powder in a suitable extraction buffer such as the buffer containing 100 mM Tris-HCl (pH 7.3) or 10 mM DTT, ultra-filtrating the obtained suspension, and subjecting the obtained specimen to the above-described determination method (Plant Physiol. 99: 1520-1525). GGT activity localized in peroxisome can be determined by isolating the peroxisomes by an ordinary method (Plant Physiol. 43: 705-713, J. Biol. Chem. 243: 5179-5184, Plant Physiol. 49: 249-251 or the like) and then determining the activity by the above-described method. These methods are well known in the art.

The resulted plants may be estimated for glutamate content. Glutamate content can be determined by, for example, pulverizing the plant body or a part thereof, obtaining an extract therefrom and examining the extract with an ordinary amino acid analyzer. For example, amino acids can be extracted by adding 500 µl of 80 % ethanol to a sample (a plant body or a part thereof), pulverizing the sample with a cell blender MM 300 (QIAGEN) and treating the obtained product at 80°C for 10 minutes. The product is centrifuged and then vacuum-revolved. The remaining sample is dissolved in 0.02 N HCl to obtain an analysis sample. The sample is passed through a 0.22 µm filter to remove impurities. In the amino acid analysis, amino acid content can be determined with amino acid analyzer LS-8800 (HITACHI). Glutamate content of a plant body can be determined on the basis of an increasing rate of glutamate content in the total amino acid content, glutamate content per a fresh weight or glutamate content per a specified tissue, preferably photosynthetic tissue such as leaf, as compared with those of the wild-type plant grown under the same conditions, and optionally, glutamate content of the plant body may be statistically treated. When the increase in glutamate. content is statistically significant in at least one of these indexes (for example, when the increase is statistically significant on a significant level of 5 % (p<0.05)), it may be considered that glutamate content is significantly increased as compared with that of the wild type plant grown under the same conditions.

By the method of the present invention, glutamate content (relative content of glutamate to the total amino acid content) of the plant body is increased to at least about 1.2 times, usually about 1.2 to 2.5 times as high as that of the wild type plant body cultivated under the same conditions; and glutamate content per fresh weight is increased to at least about 1.2 times, usually about 1.5 to 3 times.

After the transformed plant having increased glutamate content is identified, it is possible to examine whether the characteristics thereof can be genetically stably kept or not. For this purpose, plants are cultivated under an ordinary light condition, the seeds are taken from them and the character and separation of the descendants thereof are analyzed. When the inhibition of the growth of the plant is extreme under ordinary light conditions, the plant may be grown under a weak light condition of, for example, about 30 µmol m⁻² s⁻¹ or under a condition of a high CO₂ concentration of, for example, 0.7 % and an ordinary light condition, and then the characters thereof can be analyzed. The presence or absence of the introduced nucleic acid constructs, the position thereof and the expression thereof in the progenies can be analyzed in the same manner as that of the primary transformants.

The transformants thus having increased glutamate content are either heterozygous or homozygous as for the sequence derived from the nucleic acid constructs integrated into the genomes or as for the disrupted genes. If necessary, either heterozygotes or homozygotes can be obtained by, for example, cross-fertilization. The sequence derived from the nucleic acid constructs integrated into the genomes segregates according to Mendel's law in the progenies. Therefore, for attaining the object of the present invention, it is preferred to use homozygous plants from the viewpoint of the stability of the characters. Although the plants of the present invention can be grown under ordinary cultivation conditions, it is desirable to grow them under as strong as possible light so far as the growth inhibition does not occur.

In the production of the seeds of the present invention, it is particularly preferred to cultivate the homozygous plants and harvest the seeds thereof. The homozygous plants can be selected by repeating the cultivation of the generations until the interested phenotypes do not separate or, in other words, the homozygous plants can be selected by selecting a line exhibiting the interested phenotype in all the progenies. The homozygotes can be selected by PCR or Southern analysis. When the object of the present invention has been attained by inserting a transposon or T-DNA, such molecular biological techniques are useful.

The seeds of the present invention can be obtained by cultivating a plant wherein GGT activity is lacked or reduced, or in particular, a plant confirmed to have an increased glutamate content, which is obtained by the above-described method, under ordinary conditions and then collecting the seeds thereof. For example, a plant which is homozygous for the disrupted GGT gene is cultivated under ordinary conditions and the seeds thereof are collected. Preferably, the plant of the present invention is cultivated under a light condition of about 30 to 70 µmol⁻² s⁻¹ and the seeds thereof are collected to obtain the seeds of the present invention. By determining glutamate content of the plant by the above-described method, the seeds of the present invention can be confirmed to have glutamate content higher than that of the seeds of a corresponding wild-type plant cultivated under the same conditions.

The plants and seeds of the present invention are usable as foods and food materials in the same manner as those of corresponding wild-type plants. Therefore, the plants and seeds of the present invention are usable as foods directly or after cooking or processing by an ordinary method. Particularly preferred foods are those which are desired to have a taste enhanced by glutamate, such as soy sauce, *miso* (fermented soybean paste), tomato ketchup, *natto* (fermented soybeans), soups and snacks.

The following Examples will further illustrate the method for obtaining the plants of the present invention by screening the gene destruction plant library of Arabidopsis thaliana and also the characteristics of the obtained plant and seeds. It will be apparent for those skilled in the art that the plants of the present invention, their seeds and the method of the present invention are not limited to the particular plant, Arabidopsis thaliana.

### Examples

### Example 1 Acquisition of GGT-defective line of Arabidopsis thaliana

### (1) Cultivation of plants

PNS (Mol. Gen. Genet. 204: 430-434) or MS (Physiol Plant 15: 473-479) inorganic salts containing 1 % (w/v) of sucrose, 0.05 % (w/v) of MES [2-(N-morpholino) ethanesulfonic acid] and 0.8 % (w/v) of agar were used as the basal medium for plates. In the cultivation on rock wools, only PNS inorganic salts were used as source of nutrient.

### (2) Preparation of primers for screening GGT-defective lines

AlaAT genes were obtained on the basis of information of alanine aminotransferase (AlaAT) genes of Arabidopsis thaliana. AlaAT genes are also referred to as GGT genes.

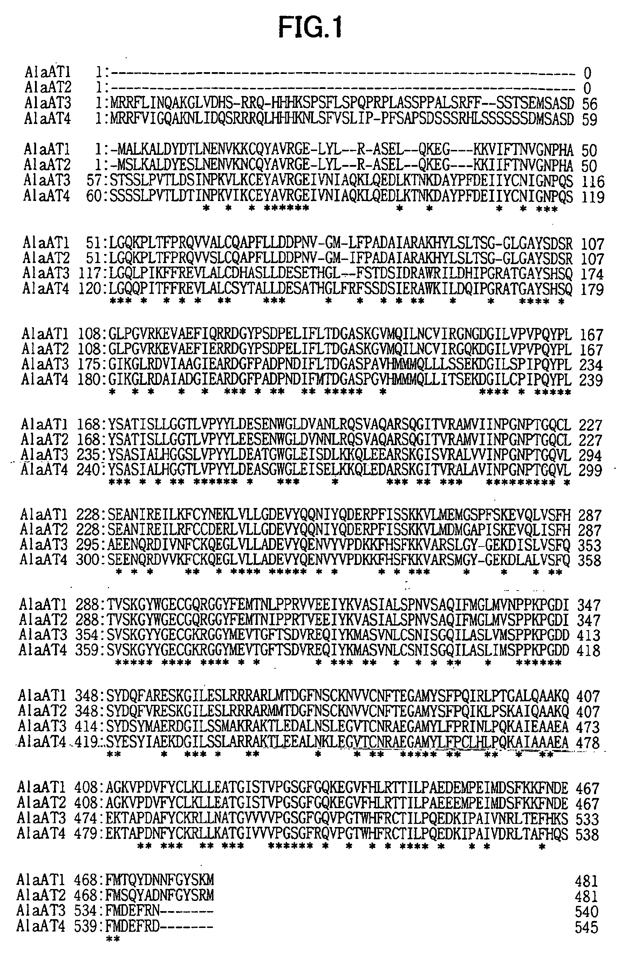

The copy number and sequence of AlaAT are estimated from data published on Internets and the primers were prepared. According to the data retrieval using "Alanine aminotransferase" and "Arabidopsis" as the key words, it was found that at least 4 copies of genes supposed to be alanine aminotransferase were present on the genome. Genbank accession numbers of the respective genes were AC005292 (F26F24.16), AC011663 (F5A18.24), AC016529 (T10D10.20) and AC026479 (T13M22.3). The genes were named AlaAT1, 2, 3 and 4, respectively. The comparisons of the deduced amino acid sequences are shown in Fig. 1. According to EST information, the amount of the expression of AlaAT1 was supposed to be highest among the 4 copies and, accordingly, the effect of the gene disruption was expected to be most remarkable. PCR primers for screening the gene disruption lines were prepared according to the AlaAT1 sequence (Table 1). These primers were designed according to the system provided by Kazusa DNA Laboratory.

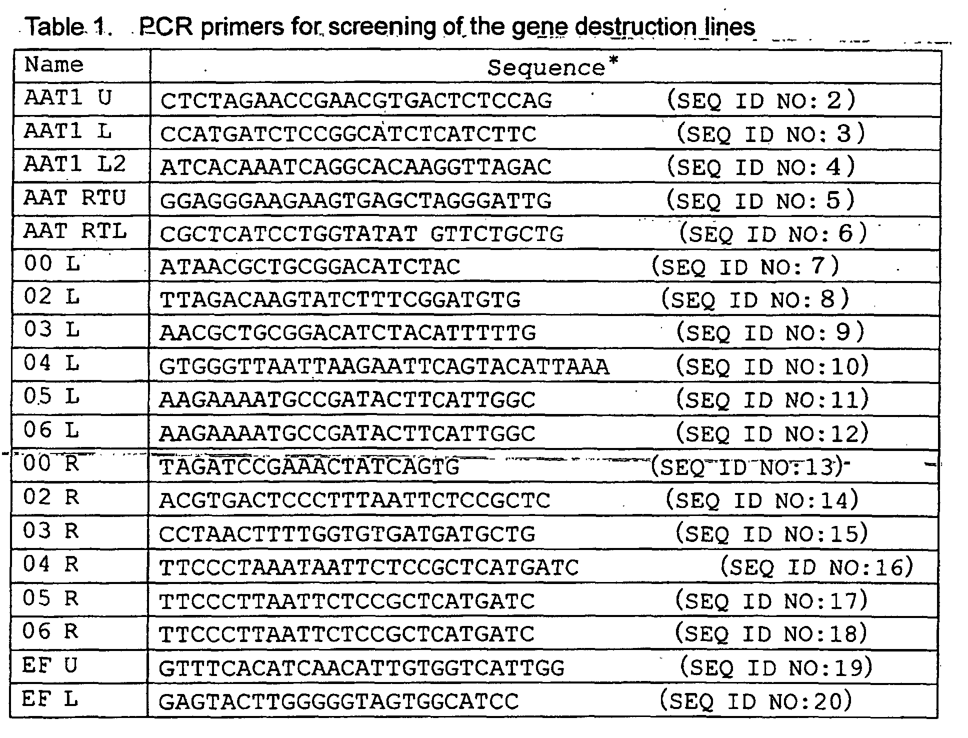

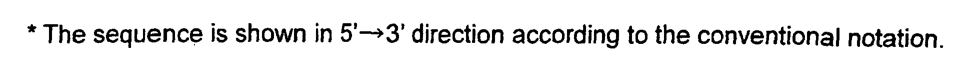

### (3) Isolation of AlaAT destruction lines

The screening for AlaAT in the gene disruption Arabidopsis thaliana Library was performed according to a system provided by Kazusa DNA Laboratory. The screening was conducted by the procedure described in 2-4-c in Plant Cell Engineering Series 14 *"Shokubutsu no Genome Kenkyu Protocol* (Protocol of Study of Plant Genome)" (published by Shujunsha).

In the primary screening, (AAT1U/AAT1L) was used as the primer of the gene side, and (00L/02L/03L/04L/05L/06L/00R/02R/03R/04R/05R/06R) were used as the tag primers in the respective, corresponding pools. The relationship between the tag primers used and the respective pools is shown in Table 2.

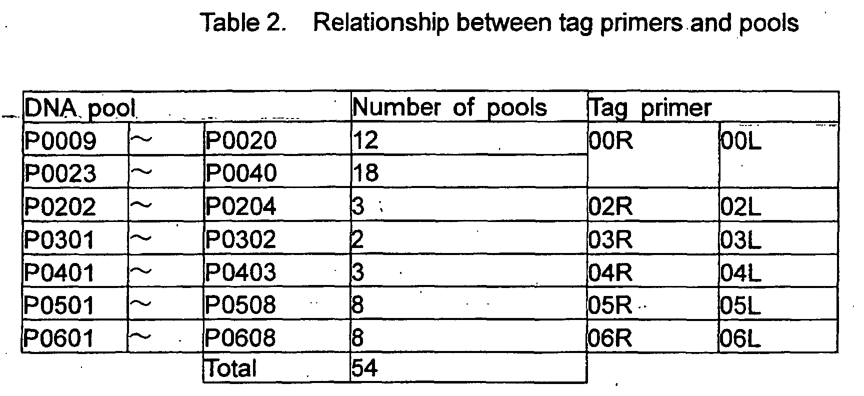

The polymerase used was EX-taq (TAKARA). 20 µl of the reaction solution contains about 38.4 ng (about 100 pg x 384) of template DNA, 10 pmol of tag primer, 10 pmol of primer for the gene, 2 µl of 10 x buffer, 5 nmol of dNTP and 0.5 U of Ex-taq. PCR cycle comprised 94°C for 45 seconds, 52°C for 45 seconds and 72°C for 3 minutes. After the repetition for 35 cycles, 10 µl of the PCR product was separated by the electrophoresis with 1 % agarose gel. The amplified DNA fragments were observed after EtBr staining. The gel was denatured by the immersion in a denaturing solution (1.5 M NaCl, 0.5 M NaOH) for 20 minutes. The gel was then immersed in a neutralizing solution [0.5 M Tris-HCl (pH 8.0), 1.5 M NaCl] for 20 minutes. After blotting onto membrane-Hybond N+ (Amersham Pharmacia Biotech) with 20 x SSC (3M NaCl, 0.3 M sodium citrate), DNA was fixed on the membrane by UV cross-linking. The hybridization and detection were conducted with AlkPhos-Direct DNA detection kit (Amersham Pharmacia Biotech) according to the protocol attached thereto. The hybridization temperature was 65°C. PCR was conducted using AATIU/AAT1 L as probes and genome DNA as a template. The amplified fragments were purified with GFX PCR DNA and Gel Band purification kit (Amercham Pharmacia Biotech).

In the primary screening, a mixture of genome DNA extracted from 384 independent tag-inserted lines was taken as one pool. 54 pools (384 x 54 = 20736 lines) were subjected to PCR. The amplification products were subjected to Southern analysis to confirm whether the intended product was amplified or not. Pool P0035 having positive results in the primary screening was subjected to the secondary screening. The primer combination for PCR for the secondary screening was AAT1U/00L and AAT1L/00L which gave positive results in the primary screening. By the secondary screening, it was made apparent that AlaAT1 tag was inserted in one line, line 8046.

### (4) Determination of the location of tag insertion

DNA extracted from the determined tag-inserted line was used as the template. PCR was conducted by using two primer sets (AAT1U/00L, AAT1L/OOL). The amplified fragments were cloned to obtain pGEM T-easy vector (Promega). For the sequencing, a DNA sequencer, ABI PRISMTM 377 DNA sequencer (PERKIN ELMER) was used.

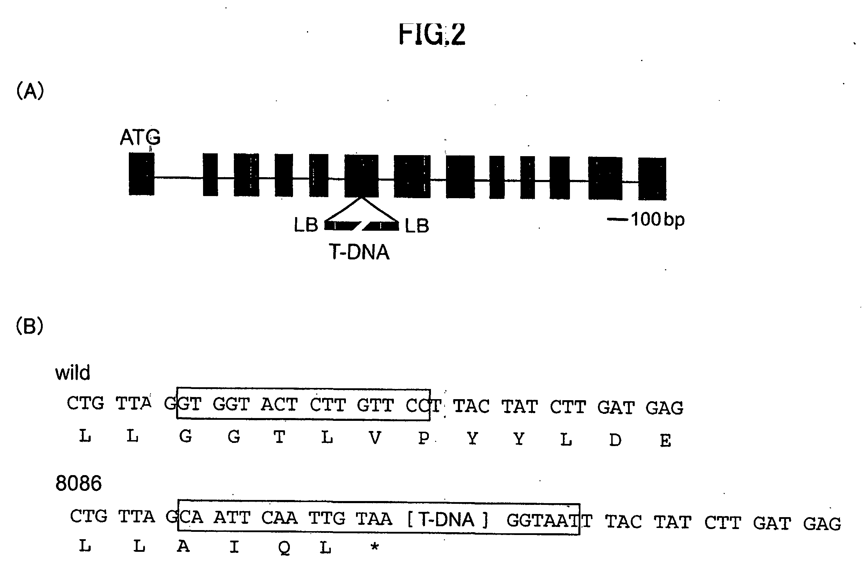

It was found that the tag was inserted in the sixth exon with the deletion of 16bp and that 176-GGTLV-180 was replaced with 176-AIQL (end)-180 by the insertion of the tag. The determined position of tag insertion and the neighboring sequences are shown in Fig. 2.

### Example 2 Analysis of characteristics of GGT-defective line

### (1) Selection of homozygotes

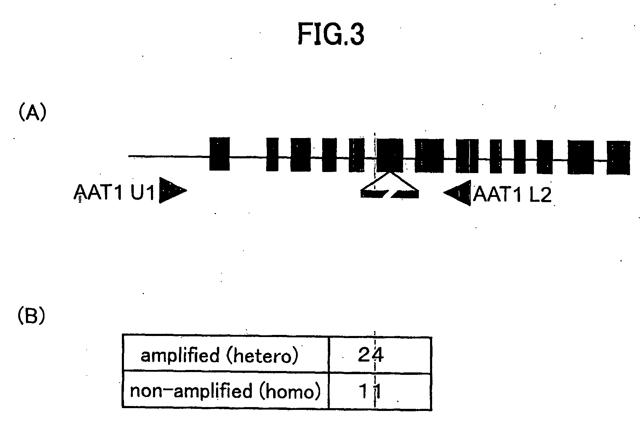

T2 seeds of the line of which the tag insertion had been confirmed were placed on MS medium containing 10 mg/l of hygromycin. Three weeks later, the seedlings thus obtained were transplanted in rock wool, and DNA was extracted from about 5 mm x 5 mm samples of rosette leaves. The extraction was conducted according to Li method (Plant J. 8: 457 to 463). For the identification of the homozygotes, PCR was conducted with primers (AAT1U/AAT1L2) flanking the tag. 30 cycles of PCR were conducted, wherein denaturation was conducted at 94°C for 30 seconds, the annealing was conducted at 57°C for 30 seconds and the elongation was conducted at 72°C for 60 seconds. For the control, wild type genome DNA was used as the template. An aliquot of the PCR product was separated on 1 % agarose gel by electrophoresis. Homozygotes were found in 11 lines in total 35 lines (Fig. 3).

### (2) Detection of GGT expression

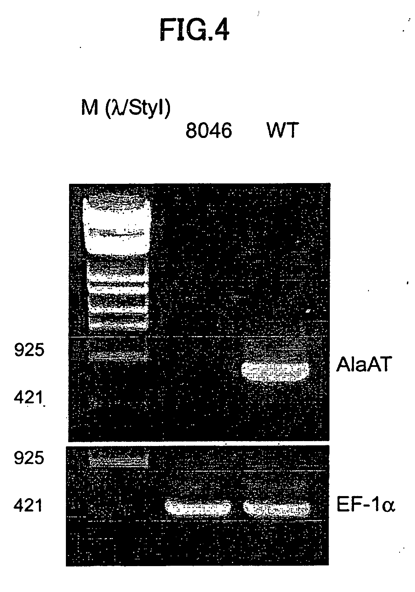

The obtained homozygous line was subjected to RT-PCR by using the progenies thereof to confirm that the gene disruption occurred. The seeds of the homozygotes were seeded on MS medium containing 10 mg/l of hygromycin, and it was confirmed that all the individuals were resistant. Total RNA was extracted from seedlings with ISOGEN (Nippon gene) two weeks after the seeding. After the treatment with DNase followed by the reverse transcription with oligo-dT primer using superscript II (GIBCO), PCR was conducted with primers (AAT1 RTU / AAT1RTL) flanking the tag using the synthesized single-strand cDNA as the template. 28 cycles of PCR were conducted, wherein denaturation was conducted at 94°C for 30 seconds, the annealing was conducted at 57°C for 30 seconds and the elongation was conducted at 72°C for 60 seconds. For the control, EF1-α (EFU/EFL) was used. An aliquot of the PCR product was separated on 1 % agarose gel by electrophoresis. No full-length mRNA for AlaAT1 was found in the tag-inserted line (Fig. 4). According to these results, the tag-inserted line was named "aat1-1" and used for the following analysis.

### (3) Effect of light intensity on tag-inserted line aat1-1:

The seeds of tag-inserted line aat1-1 were planted in the soil, and cultivated under an ordinary light strength condition (about 70 µmol m⁻²s⁻¹) in a light period of 16 hours and dark period of 8 hours.

The aboveground parts of 20 seedlings obtained under the above-described conditions were taken, and the weight of them was determined repeatedly three times. The average of them was calculated. It was found that no significant difference was found in the growth under the weak light condition (about 30 µmol m⁻² s⁻¹), though the growth was seriously inhibited under the ordinary light strength condition (Table 3). These results suggested that aat1-1 was damaged by the photo inhibition which is caused by the incomplete photorespiration.

**Table 3**

| Effect of light intensity | | |
|---|---|---|
| Relative weight | | |
| Light intensity (µmol m⁻²s⁻¹) | Control | aat1-1 |
| 30 | 1 | 0.89 |
| 100 | 1 | 0.34 |

### (4) Enzymatic activity of tag-inserted line aat1-1

For determining the enzymatic activity, the protein was extracted from seedlings grown under a light condition of 70µmol m⁻² s⁻¹ for 2 weeks after seeding on PNS medium. The plant (fresh weight: about 200 mg) was frozen in liquid nitrogen and then the tissue thereof was crushed by using a mortar and a pestle. 1 ml of the extract solution [100 mM Tris-HCl (pH 7.3), 10 mM DTT] was added thereto, and the obtained mixture was centrifuged at 15,000 rpm for 10 minutes to remove insoluble matters. This process was repeated 3 times. The desalting treatment was conducted with a filter UFV5BGCOO (Millipore) for the ultrafiltration. 0.5 ml of the extract was concentrated to a concentration of 10 times by the centrifugation at 10,000 rpm for about 45 minutes. After the dilution to a concentration of 1/10 with the extract, the same process was repeated 3 times. The protein concentration was determined with a protein assay kit (Bio-Rad). The extract containing 10 % glycerol was added thereto so as to obtain a final concentration of 2 mg per 1 ml of extract to obtain the crude extract, which was used for the subsequent determination of the enzyme activity.

The activity of the reaction of Ala (alanine) + αKG (α ketoglutarate) → Glu (glutamate) + Pyr (pyruvate) was determined as the change of OD at 340 nm by coupling the reaction with the oxidation reaction of NADH with LDH (EC 1.1.1.27). For the reaction, 10 mg of the crude extract was used for 600 µl of the reaction solution [100 mM Tris-HCl (pH 7.3), 100 mM Ala, 0.11 mM pyridoxal 5-phosphate, 0.11 mM NADH, 15 mM αKG, 80 mM NH₄Cl, 1200 U/I LDH (SIGMA L2375)].

The reactivity in the reaction of Glu + Pyr → Ala + α KG and Glu + glyoxylate → glycine (Gly) + α KG was determined as the change of OD at 340nm by coupling the reaction with the oxidation reaction of NADH with NAD⁺-GDH (EC 1.4.1.3). For the reaction, 10 mg of the crude extract was used for 600 µl of the reaction solution [100 mM Tris-HCl (pH 7.3), 100 mM Glu, 0.11 mM pyridoxal 5-phosphate, 0.11 mM NADH, 15 mM Pyr, 1200 U/l GDH (G2501)]. When glyoxylate was used as the amino acceptor, the same reaction solution was used except that Pyr was replaced with glyoxylate.

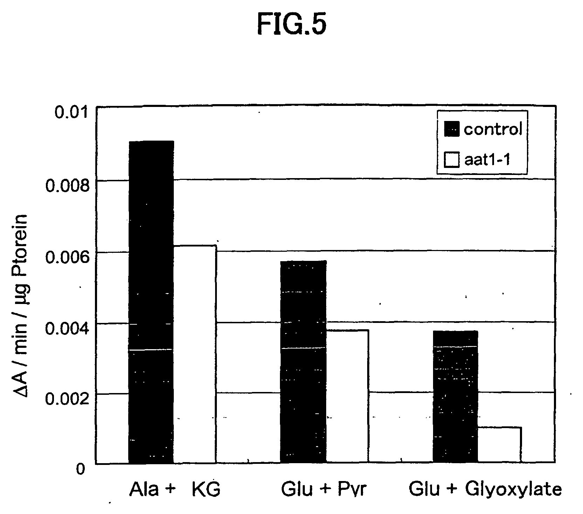

The respective reaction activities are shown in Fig. 5. It was found that in all of the three reactions which are considered to be catalyzed by AlaAT, the reactivity in aat1-1 was reduced. It was also found that the activity of catalyzing the reaction for transferring amino group from glutamate to glyoxylate, namely, GGT activity, was remarkably reduced in aat1-1.

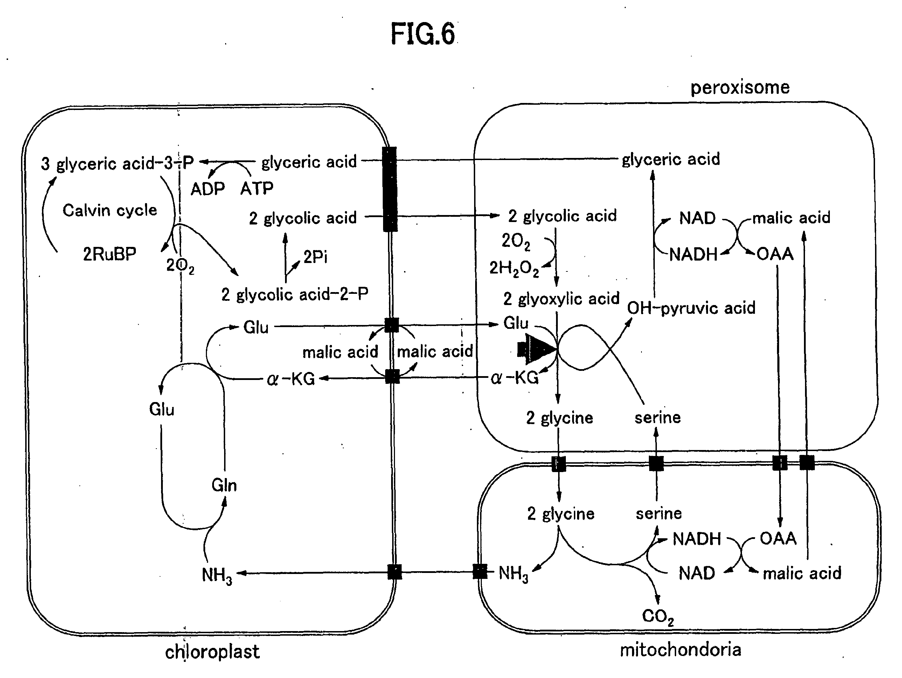

Thus, from the combination of the growth inhibition under the strong light condition with the results of the determination of the enzyme activity, it was found that under physiological conditions, AlaAT1 is greatly involved in the reaction for synthesizing glycine from glyoxylate in the glycolate pathway which is the actual photorespiration pathway (refer to the arrow in Fig. 6).

### (5) Amino acid analysis of tag-inserted aat1-1

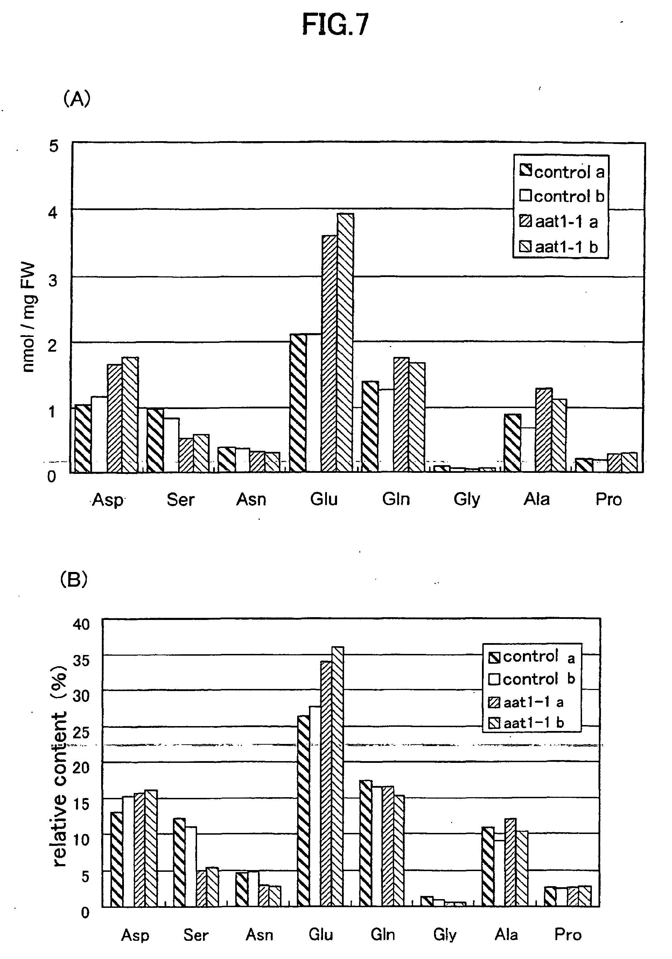

For determining the free amino acids content, amino acids were extracted from seedlings obtained by growing under the light condition of 70 µmol m⁻² s⁻¹ for 2 weeks after seeding on PNS medium. The plant (fresh weight: about 40 mg) was frozen in liquid nitrogen and then stored at -80°C. To the frozen sample, 500 µl of 80 % ethanol was added. The tissue was crushed with a cell crusher MM 300 (QIAGEN) and then treated at 80°C for 10 minutes to extract amino acids. After the centrifugation conducted at 15,000 rpm for 10 minutes, the supernatant was taken. 500 µl of 80 % ethanol at 80°C was added to the obtained precipitate, and the mixture was thoroughly stirred and then treated at 80°C for further 10 minutes. After the centrifugation at 15,000 rpm for 10 minutes, the supernatant was taken as the amino acid extract. 1 ml of the amino acid extract was rotated under reduced pressure to completely remove ethanol and water. 300 µl of 0.02 N HCl was added to the remaining sample. After vortexing followed by centrifugation, the supernatant was taken. The impurities were removed by passing through a 0.22 µm filter to obtain the sample for analysis. The amino acid analysis was conducted with an amino acid analyzer LS-8800 (HITACHI). The content of major amino acids (nmol/mg FW) and the relative amounts thereof to the total amount of the amino acids are shown in Fig. 7. The results of the analysis showed that the accumulated amount of glutamate was increased in aat1-1 (Fig. 7).

### <Sequence listing free text>

SEQ ID NO: 2 to NO: 20: PCR primers

### Example 3 Determination of amino acid content in seeds of tag-inserted line aat1-1

### (1) Cultivation of aat1-1 plants

Seeds of tag-inserted line aat1-1 and control plant Co1-0 were seeded on PNS solid medium and cultivated under the light condition (70 µmol m⁻² s⁻¹) and a light period of 16 hours and dark period of 8 hours for 3 weeks, and then transplanted in a rock wool. The rock wool in which plant bodies Co1-0 were planted and the rock wool in which plants aat-1 were planted were placed on the same tray. PNS solution was given to them as a fertilizer once a week. The seeds were collected and were analyzed for amino acids after thoroughly dried in a desiccator. Then the amino acid analysis was conducted.

### (2) Analysis of amino acids in seeds of aat1-1

About 10 mg of dry seeds and 500 µl of 80 % ethanol were transferred into 2 ml tube, and the seeds were thoroughly crushed with a cell crusher MM 300 (QIAGEN) and then treated at 80°C for 10 minutes to extract amino acids. After centrifugation at 15,000 rpm for 10 minutes, the supernatant was removed. 500 µl of 80 % ethanol was added to the obtained precipitate, and the mixture was thoroughly stirred and then treated at 80°C for 10 minutes. After centrifugation at 15,000 rpm for 10 minutes, the supernatant was taken as the amino acid extract sample.

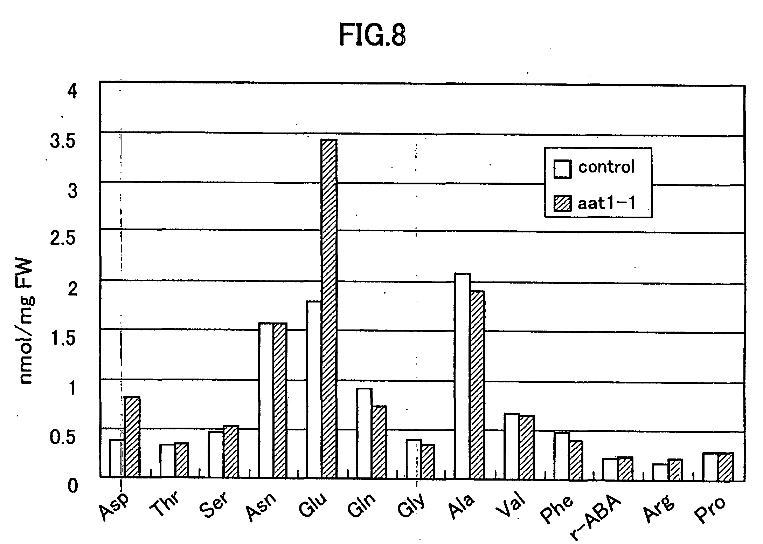

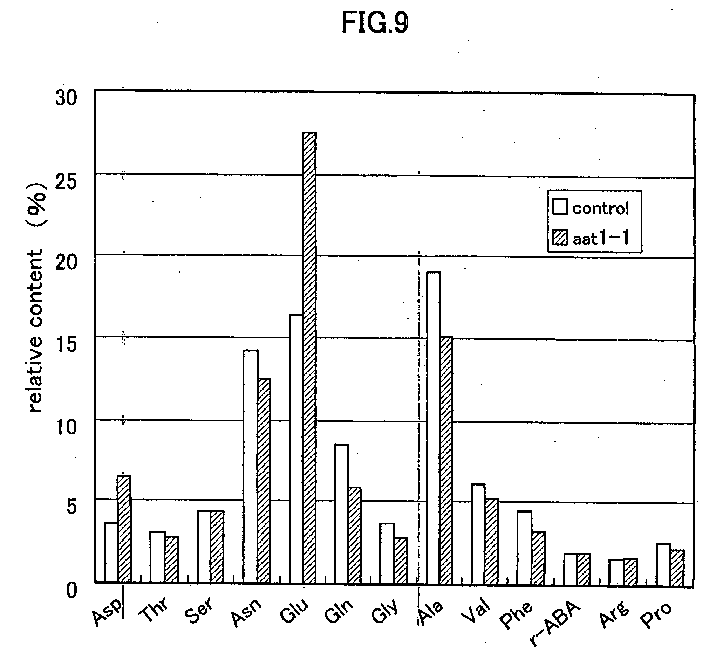

One ml of the amino acid extract was rotated under reduced pressure to completely remove ethanol and water. Five hundred µl of sterilized water and 500 µl of diethyl ether were added to the sample thus dried to solid, and they were thoroughly stirred. After the centrifugation conducted at 15,000 rpm for 5 minutes, the upper layer (ether phase) was removed. The aqueous phase was centrifuged under reduced pressure to completely remove water. Three hundred µl of 0.02 N HCl was added to the exsiccated sample. The sample was vortexed and centrifuged and supernatant was recovered. The impurities were removed by passing the supernatant through 0.22 µm filter to obtain the sample for the amino acid analysis. The amino acid was analyzed with an amino acid analyzer LS-8800 (HITACHI). The major amino acids contents (nmol/mg FW) and the relative amounts (%) thereof to the total amount of the amino acids are shown in Figs. 8 and 9, respectively.

The results of the analysis showed that glutamate content of the seeds was increased.

According to the present invention, glutamate content of plants and/or seeds of them can be increased and, therefore, the plants and/or seeds having an increased glutamate content can be obtained. Thus, according to the present invention, it is made possible to increase glutamate content of edible plant bodies and also that of the whole fruits thereof, such as spinach, cabbages, lettuces and tomatoes, and glutamate content of edible seeds of plants such as soybeans, adzuki beans, broad beans, kidney beans and peas. As a result, the taste of these plants and/or seeds can be improved. Further, the taste of foods prepared from these plant bodies and seeds can be improved.

## Claims

1. A plant wherein the activity of glutamate glyoxylate aminotransferase is lacked or reduced.

2. The plant according to claim 1, wherein the activity of glutamate glyoxylate aminotransferase is lacked or reduced, and wherein the glutamate content of said plant has increased compared to the corresponding wild type plant which is cultivated under the same condition.

3. The plant according to claim 1, wherein the activity of glutamate glyoxylate aminotransferase is lacked or reduced, and wherein the glutamate content of said plant is not less than 1.2 fold higher than the corresponding wild type plant which is cultivated under the same condition.

4. The plant according to claim 1, wherein a function of a gene encoding a protein having the activity of glutamate glyoxylate aminotransferase is inhibited.

5. The plant according to claim 4, wherein a gene encoding the protein having the activity of glutamate glyoxylate aminotransferase is disrupted.

6. The plant according to claim 4, wherein the expression of a gene encoding the protein having the activity of glutamate glyoxylate aminotransferase is inhibited.

7. The plant according to any one of claims 1 to 6, wherein the activity of glutamate glyoxylate aminotransferase is the activity in peroxisomes.

8. The plant according to claim 7, wherein the activity of glutamate glyoxylate aminotransferase is the activity in peroxisomes in photosynthetic tissues.

9. The plant according to any one of claims 4 to 6, wherein the protein having the activity of glutamate glyoxylate aminotransferase has the amino acid sequence of amino acid residue no.1 to amino acid residue no.478 in the sequence SEQ ID NO: 1.

10. The plant according to any one of claims 4 to 6, wherein the protein having the activity of glutamate glyoxylate aminotransferase has the amino acid sequence of amino acid residue no.1 to amino acid residue no.481 in the sequence SEQ ID NO: 1.

11. A seed of the plant according to any one of claims 1 to 10.

12. The seed according to claim 11, wherein the glutamate content is increased compared to a seed of the corresponding wild type plant which is cultivated under the same condition.

13. The seed according to claim 11, wherein the glutamate content is increased less than 1.2 fold compared to a seed of the corresponding wild type plant which is cultivated under the same condition .

14. A method of increasing the glutamate content in a plant and/or a seed compared to the corresponding wild type plant which is cultivated under the same condition, comprising the step of causing a defect or a reduction in the activity of glutamate glyoxylate transferase.

15. The method according to claim 14, wherein a function of a gene encoding a protein having the activity of glutamate glyoxylate aminotransferase is inhibited.

16. The method according to claim 15, wherein a gene encoding the protein having the activity of glutamate glyoxylate aminotransferase is disrupted.

17. The method according to claim 15, wherein an expression of a gene encoding the protein having the activity of glutamate glyoxylate aminotransferase is inhibited.

18. The method according to any one of claims 14 to 17, wherein the activity of glutamate glyoxylate aminotransferase is the activity in peroxisomes.

19. The method according to claim 18, wherein the activity of glutamate glyoxylate aminotransferase is the activity in peroxisomes in photosynthetic tissues.

20. The method according to any one of claims 14 to 17, wherein the protein having the activity of glutamate glyoxylate aminotransferase has the amino acid sequence of amino acid residue no.1 to.478 in the sequence SEQ ID NO: 1.

21. The method according to any one of claims 14 to 17, wherein the protein having the activity of glutamate glyoxylate aminotransferase has the amino acid sequence of amino acid residue no.1 to 481 in the sequence SEQ ID NO: 1.

22. A method of producing a seed having increased glutamate content compared to a seed obtained from the corresponding wild type plant which is cultivated under the same condition, comprising the steps of cultivating the plant according to any one of claims 1 to 10 and harvesting seed from said plant.

23. Use of the plant according to anyone of claims 1 to 10 for producing a food.

24. A food containing the plant according to any one of claims 1 to 10.

25. Use of the seed according to any one of claims 11 to 13 for producing a food.

26. A food containing the seed according to any one of claims 11 to 13.
